# EUROPEAN PATENT APPLICATION

(11) **EP 1 619 246 A1**
(43) Date of publication of application: **25.01.2006**
(21) Application number: 04291879.7
(22) Date of filing: 23.07.2004
(51) Int. Cl.: C12N 9/12, C07K 14/165, G01N 33/68, C12Q 1/70

(54) **RNA dependent RNA polymerases from coronavirus and their use in molecular biology and drug screening**

(71) Applicant: Université de la Méditerranée, Aix-Marseille II, 13284 Marseille Cedex 07 (FR)
(72) Inventor: Imbert, Isabelle, 13009 Marseille (FR); Guillemot, Jean-Claude, 13005 Marseille (FR); Canard, Bruno, 13260 Cassis (FR); Ferron, François-Patrice, 13006 Marseille (FR)
(74) Representative: Breese, Pierre

(57) **Abstract**

The present invention concerns a RNA dependent RNA polymerase the amino acid sequence of which presents at least 60% similarity with the following sequence : 
AIASEFSSLPSYAAYATAQEAYEQAVANGDSEVVLKKLKKSLNVAKSEFDRDAAMQRKLEK
MADQAMTQMYKQARSEDKRAKVTSAMQTMLFTMLRKLDNDALNNI INNARDGCVPLNI IPL
TTAAKLMVVVPDYGTYKNTCDGNTFTYASALWEIQQVVDADSKIVQLSEINMDNSPNLAWP
LIVTALRANSAVKLQ (SEQ ID NO. 1 in the sequence listing in
appendix)
 and fragments thereof. The present invention also concerns its use as a biotech reagent and/or in a method for screening biologically active compound which can be used as antiviral agent.

SEQUENCE LISTING

<110> Université de la Méditerranée

<120> RNA dependent RNA polymerases from coronavirus and their use in
molecular biology and drug screening

<130> 37104/EP

<140> EP XXXXXX
<141> 2004-07-23

<160> 8

<170> PatentIn version 3.1

<210> 1
<211> 198
<212> PRT
<213> Coronavirus

<220>
<221> PEPTIDE
<222> (1)..(198)
<223> ORF1ab polyprotein SARS coronavirus - isolate TOR2

<400> 1

Ala Ile Ala Ser Glu Phe Ser Ser Leu Pro Ser Tyr Ala Ala Tyr Ala
1               5                   10                  15

Thr Ala Gln Glu Ala Tyr Glu Gln Ala Val Ala Asn Gly Asp Ser Glu
            20                  25                  30

Val Val Leu Lys Lys Leu Lys Lys Ser Leu Asn Val Ala Lys Ser Glu
        35                  40                  45

Phe Asp Arg Asp Ala Ala Met Gln Arg Lys Leu Glu Lys Met Ala Asp
    50                  55                  60

Gln Ala Met Thr Gln Met Tyr Lys Gln Ala Arg Ser Glu Asp Lys Arg
65                  70                  75                  80

Ala Lys Val Thr Ser Ala Met Gln Thr Met Leu Phe Thr Met Leu Arg
                85                  90                  95

Lys Leu Asp Asn Asp Ala Leu Asn Asn Ile Ile Asn Asn Ala Arg Asp
            100                 105                 110

Gly Cys Val Pro Leu Asn Ile Ile Pro Leu Thr Thr Ala Ala Lys Leu
        115                 120                 125

Met Val Val Val Pro Asp Tyr Gly Thr Tyr Lys Asn Thr Cys Asp Gly
    130                 135                 140

Asn Thr Phe Thr Tyr Ala Ser Ala Leu Trp Glu Ile Gln Gln Val Val
145                 150                 155                 160

Asp Ala Asp Ser Lys Ile Val Gln Leu Ser Glu Ile Asn Met Asp Asn
                165                 170                 175

Ser Pro Asn Leu Ala Trp Pro Leu Ile Val Thr Ala Leu Arg Ala Asn

180                 185                 190

Ser Ala Val Lys Leu Gln
        195



<210>  2
<211>  210
<212>  PRT
<213>  Coronavirus

<220>
<221>  PEPTIDE
<222>  (1)..(210)
<223>  ORF1ab polyprotein Avian infectious bronchitis virus (Beaudette)

<400>  2

Ser Val Thr Gln Glu Phe Ser His Ile Pro Ser Tyr Ala Glu Tyr Glu
1               5                   10                  15

Arg Ala Lys Asn Leu Tyr Glu Lys Val Leu Val Asp Ser Lys Asn Gly
            20                  25                  30

Gly Val Thr Gln Gln Glu Leu Ala Ala Tyr Arg Lys Ala Ala Asn Ile
        35                  40                  45

Ala Lys Ser Val Phe Asp Arg Asp Leu Ala Val Gln Lys Lys Leu Asp
    50                  55                  60

Ser Met Ala Glu Arg Ala Met Thr Thr Met Tyr Lys Glu Ala Arg Val
65                  70                  75                  80

Thr Asp Arg Arg Ala Lys Leu Val Ser Ser Leu His Ala Leu Leu Phe
                85                  90                  95

Ser Met Leu Lys Lys Ile Asp Ser Glu Lys Leu Asn Val Leu Phe Asp
            100                 105                 110

Gln Ala Ser Ser Gly Val Val Pro Leu Ala Thr Val Pro Ile Val Cys
        115                 120                 125

Ser Asn Lys Leu Thr Leu Val Ile Pro Asp Pro Glu Thr Trp Val Lys
    130                 135                 140

Cys Val Glu Gly Val His Val Thr Tyr Ser Thr Val Val Trp Asn Ile
145                 150                 155                 160

Asp Thr Val Ile Asp Ala Asp Gly Thr Glu Leu His Pro Thr Ser Thr
                165                 170                 175

Gly Ser Gly Leu Thr Tyr Cys Ile Ser Gly Ala Asn Ile Ala Trp Pro
            180                 185                 190

Leu Lys Val Asn Leu Thr Arg Asn Gly His Asn Lys Val Asp Val Val
        195                 200                 205

Leu Gln
    210


<210>  3
<211>  197

<212>  PRT
<213>  Coronavirus

<220>
<221>  PEPTIDE
<222>  (1)..(197)
<223>  ORF1ab polyprotein Bovine coronavirus - isolate BCoV-ENT

<400>  3

Ala Leu Gln Ser Glu Phe Val Asn Met Ala Ser Phe Val Glu Tyr Glu
1               5                   10                  15

Val Ala Lys Lys Asn Leu Asp Glu Ala Arg Ser Ser Gly Ser Ala Asn
            20                  25                  30

Gln Gln Gln Leu Lys Gln Leu Glu Lys Ala Cys Asn Ile Ala Lys Ser
        35                  40                  45

Ala Tyr Glu Arg Asp Arg Ala Val Ala Arg Lys Leu Glu Arg Met Ala
    50                  55                  60

Asp Leu Ala Leu Thr Asn Met Tyr Lys Glu Ala Arg Ile Asn Asp Lys
65                  70                  75                  80

Lys Ser Lys Val Val Ser Ala Leu Gln Thr Met Leu Phe Ser Met Val
                85                  90                  95

Arg Lys Leu Asp Asn Gln Ala Leu Asn Ser Ile Leu Asp Asn Ala Val
            100                 105                 110

Lys Gly Cys Val Pro Leu Asn Ala Ile Pro Ser Leu Ala Ala Asn Thr
        115                 120                 125

Leu Thr Ile Ile Val Pro Asp Lys Ser Val Tyr Asp Gln Val Val Asp
    130                 135                 140

Asn Val Tyr Val Thr Tyr Ala Gly Asn Val Trp Gln Ile Gln Thr Ile
145                 150                 155                 160

Gln Asp Ser Asp Gly Thr Asn Lys Gln Leu Asn Glu Ile Ser Asp Asp
                165                 170                 175

Cys Asn Trp Pro Leu Val Ile Ile Ala Asn Arg His Asn Glu Val Ser
            180                 185                 190

Ala Thr Val Leu Gln
        195


<210>  4
<211>  195
<212>  PRT
<213>  Coronavirus

<220>
<221>  PEPTIDE
<222>  (1)..(195)
<223>  ORF1ab polyprotein Human coronavirus 229E

<400>  4

Ser Val Ala Ser Ser Phe Val Gly Met Pro Ser Phe Val Ala Tyr Glu

1               5                   10                  15

Thr Ala Arg Gln Glu Tyr Glu Asn Ala Val Ala Asn Gly Ser Ser Pro
            20                  25                  30

Gln Ile Ile Lys Gln Leu Lys Lys Ala Met Asn Val Ala Lys Ala Glu
        35                  40                  45

Phe Asp Arg Glu Ser Ser Val Gln Lys Lys Ile Asn Arg Met Ala Glu
    50                  55                  60

Gln Ala Ala Ala Ala Met Tyr Lys Glu Ala Arg Ala Val Asn Arg Lys
65                  70                  75                  80

Ser Lys Val Val Ser Ala Met His Ser Leu Leu Phe Gly Met Leu Arg
                85                  90                  95

Arg Leu Asp Met Ser Ser Val Asp Thr Ile Leu Asn Met Ala Arg Asn
            100                 105                 110

Gly Val Val Pro Leu Ser Val Ile Pro Ala Thr Ser Ala Ala Arg Leu
        115                 120                 125

Val Val Val Val Pro Asp His Asp Ser Phe Val Lys Met Met Val Asp
    130                 135                 140

Gly Phe Val His Tyr Ala Gly Val Val Trp Thr Leu Gln Glu Val Lys
145                 150                 155                 160

Asp Asn Asp Gly Lys Asn Val His Leu Lys Asp Val Thr Lys Glu Asn
                165                 170                 175

Gln Glu Ile Leu Val Trp Pro Leu Ile Leu Thr Cys Glu Arg Val Val
            180                 185                 190

Lys Leu Gln
        195


<210>  5
<211>  197
<212>  PRT
<213>  Coronavirus

<220>
<221>  PEPTIDE
<222>  (1)..(197)
<223>  Polyprotein 1ab Human coronavirus (ATCC VR-759) - isolate OC43

<400>  5

Ala Leu Gln Ser Glu Phe Val Asn Met Ala Ser Phe Val Glu Tyr Glu
1               5                   10                  15

Val Ala Lys Lys Asn Leu Asp Glu Ala Arg Phe Ser Gly Ser Ala Asn
            20                  25                  30

Gln Gln Gln Leu Lys Gln Leu Glu Lys Ala Cys Asn Ile Ala Lys Ser
        35                  40                  45

Ala Tyr Glu Arg Asp Arg Ala Val Ala Lys Lys Leu Glu Arg Met Ala
    50                  55                  60

Asp Leu Ala Leu Thr Asn Met Tyr Lys Glu Ala Arg Ile Asn Asp Lys
65                  70                  75                  80

Lys Ser Lys Val Val Ser Ala Leu Gln Thr Met Leu Phe Ser Met Val
                85                  90                  95

Arg Lys Leu Asp Asn Gln Ala Leu Asn Ser Ile Leu Asp Asn Ala Val
            100                 105                 110

Lys Gly Cys Val Pro Leu Asn Ala Ile Pro Ser Leu Ala Ala Asn Thr
        115                 120                 125

Leu Asn Ile Ile Val Pro Asp Lys Ser Val Tyr Asp Gln Val Val Asp
    130                 135                 140

Asn Val Tyr Val Thr Tyr Ala Gly Asn Val Trp Gln Ile Gln Thr Ile
145                 150                 155                 160

Gln Asp Ser Asp Gly Thr Asn Lys Gln Leu Asn Glu Ile Ser Asp Asp
                165                 170                 175

Cys Asn Trp Pro Leu Val Ile Ile Ala Asn Arg Tyr Asn Glu Val Ser
            180                 185                 190

Ala Thr Val Leu Gln
        195

<210>  6
<211>  194
<212>  PRT
<213>  Coronavirus

<220>
<221>  PEPTIDE
<222>  (1)..(194)
<223>  ORF1ab polyprotein Murine hepatitis virus (MHV-A59)

<400>  6

Ser Glu Phe Val Asn Met Ala Ser Phe Val Glu Tyr Glu Leu Ala Lys
1               5                   10                  15

Lys Asn Leu Asp Glu Ala Lys Ala Ser Gly Ser Ala Asn Gln Gln Gln
            20                  25                  30

Ile Lys Gln Leu Glu Lys Ala Cys Asn Ile Ala Lys Ser Ala Tyr Glu
        35                  40                  45

Arg Asp Arg Ala Val Ala Arg Lys Leu Glu Arg Met Ala Asp Leu Ala
    50                  55                  60

Leu Thr Asn Met Tyr Lys Glu Ala Arg Ile Asn Asp Lys Lys Ser Lys
65                  70                  75                  80

Val Val Ser Ala Leu Gln Thr Met Leu Phe Ser Met Val Arg Lys Leu
                85                  90                  95

Asp Asn Gln Ala Leu Asn Ser Ile Leu Asp Asn Ala Val Lys Gly Cys
            100                 105                 110

Val Pro Leu Asn Ala Ile Pro Ser Leu Thr Ser Asn Thr Leu Thr Ile
        115                 120                 125

Ile Val Pro Asp Lys Gln Val Phe Asp Gln Val Val Asp Asn Val Tyr
    130                 135                 140

Val Thr Tyr Ala Gly Asn Val Trp His Ile Gln Phe Ile Gln Asp Ala
145                 150                 155                 160

Asp Gly Ala Val Lys Gln Leu Asn Glu Ile Asp Val Asn Ser Thr Trp
                165                 170                 175

Pro Leu Val Ile Ala Ala Asn Arg His Asn Glu Val Ser Thr Val Val
            180                 185                 190

Leu Gln

<210>  7
<211>  195
<212>  PRT
<213>  Coronavirus

<220>
<221>  PEPTIDE
<222>  (1)..(195)
<223>  Polyprotein Porcine epidemic diarrhea virus (CV777)

<400>  7

Ser Val Ala Ser Thr Tyr Val Gly Leu Pro Ser Tyr Val Ile Tyr Glu
1               5                   10                  15

Asn Ala Arg Gln Gln Tyr Glu Asp Ala Val Asn Asn Gly Ser Pro Pro
            20                  25                  30

Gln Leu Val Lys Gln Leu Arg His Ala Met Asn Val Ala Lys Ser Glu
        35                  40                  45

Phe Asp Arg Glu Ala Ser Thr Gln Arg Lys Leu Asp Arg Met Ala Glu
    50                  55                  60

Gln Ala Ala Ala Gln Met Tyr Lys Glu Ala Arg Ala Val Asn Arg Lys
65                  70                  75                  80

Ser Lys Val Val Ser Ala Met His Ser Leu Leu Phe Gly Met Leu Arg
                85                  90                  95

Arg Leu Asp Met Ser Ser Val Asp Thr Ile Leu Asn Leu Ala Lys Asp
            100                 105                 110

Gly Val Val Pro Leu Ser Val Ile Pro Ala Val Ser Ala Thr Lys Leu
        115                 120                 125

Asn Ile Val Thr Ser Asp Ile Asp Ser Tyr Asn Arg Ile Gln Arg Glu
    130                 135                 140

Gly Cys Val His Tyr Ala Gly Thr Ile Trp Asn Ile Ile Asp Ile Lys
145                 150                 155                 160

Asp Asn Asp Gly Lys Val Val His Val Lys Glu Val Thr Ala Gln Asn
                165                 170                 175

Ala Glu Ser Leu Ser Trp Pro Leu Val Leu Gly Cys Glu Arg Ile Val
            180                 185                 190

Lys Leu Gln
        195

<210>  8
<211>  195
<212>  PRT
<213>  Coronavirus

<220>
<221>  PEPTIDE
<222>  (1)..(195)
<223>  ORF1ab polyprotein transmissible gastroenteritis virus (Purdue) -
        isolate PUR46-MAD

<400>  8

Ser Val Ala Ser Ala Tyr Ala Ala Leu Pro Ser Trp Ile Ala Leu Glu
1               5                   10                  15

Lys Ala Arg Ala Asp Leu Glu Glu Ala Lys Lys Asn Asp Val Ser Pro
            20                  25                  30

Gln Ile Leu Lys Gln Leu Thr Lys Ala Phe Asn Ile Ala Lys Ser Asp
        35                  40                  45

Phe Glu Arg Glu Ala Ser Val Gln Lys Lys Leu Asp Lys Met Ala Glu
    50                  55                  60

Gln Ala Ala Ala Ser Met Tyr Lys Glu Ala Arg Ala Val Asp Arg Lys
65                  70                  75                  80

Ser Lys Ile Val Ser Ala Met His Ser Leu Leu Phe Gly Met Leu Lys
                85                  90                  95

Lys Leu Asp Met Ser Ser Val Asn Thr Ile Ile Asp Gln Ala Arg Asn
            100                 105                 110

Gly Val Leu Pro Leu Ser Ile Ile Pro Ala Ala Ser Ala Thr Arg Leu
        115                 120                 125

Val Val Ile Thr Pro Ser Leu Glu Val Phe Ser Lys Ile Arg Gln Glu
    130                 135                 140

Asn Asn Val His Tyr Ala Gly Ala Ile Trp Thr Ile Val Glu Val Lys
145                 150                 155                 160

Asp Ala Asn Gly Ser His Val His Leu Lys Glu Val Thr Ala Ala Asn
                165                 170                 175

Glu Leu Asn Leu Thr Trp Pro Leu Ser Ile Thr Cys Glu Arg Thr Thr
            180                 185                 190

Lys Leu Gln
        195

## Description

This invention is in the field of antiviral research and molecular biology techniques and reagents. It relates the discovery of a previously unknown RNA polymerase activity from the gene product nsp8 from the SARS CoV. The unknown gene product nsp8 is now identified as a novel type of RNA dependent RNA polymerase useful for :
- drug-screening and drug-design against the large family of Nidoviruses possessing homologous genes, gene products, and enzymatic activities. The nsp8 protein now represents a new, easily producible target for high-throughput screening of antiviral drugs.
- biotech reagent. An RNA polymerase, easily produced, with a strong in vitro activity, represents a unique tool in the field of biotech applications.

### Background

The first cases of Severe Acute Respiratory Syndrome (SARS) seem to originate from China (Guandong province in South East China).

A number of points about this new epidemic are now established:
- The most probable etiologic agent is a virus belonging to the *Coronaviridae* family and genetically different from other coronaviruses identified previously, including human coronaviruses. This new etiological agent has been named the SARS CoV.
- The virus is transmissible at least by the respiratory route and the secondary transmission is proven.
- The mortality rate in identified cases (thus, among severe respiratory syndromes) is ∼ 10%.

The SARS syndrome is a real paradigm for emerging viral pathogens. The viral strain probably infected previously an unidentified animal and crossed the species barrier to infect humans.

SARS epidemics of 2003 and 2004 have demonstrated that Coronaviruses, and especially the SARS CoV, are a threat to public health at the world level.

The incredibly rapid and wide spread of the virus in the world has highlighted the urgent need of an antiviral molecule that would be useful as a prophylactic and curative drug. However, the number of antiviral molecules that can be used to treat patients infected by RNA viruses is incredibly low. This is probably due to the fact that, in the past, the most severe infections due to RNA viruses (e. g. flaviviruses, alphaviruses, filoviruses, arenaviruses, phleboviruses, but also rotaviruses, enteroviruses and many others) occurred in developing countries. This market was not estimated to be profitable by pharmaceutical companies. The increasingly observed globalisation of infectious diseases forces to change this point of view. Accordingly it is important to search for efficient antiviral drugs for a large number of RNA viruses, and in priority against viruses transmitted by the respiratory route, because they have the highest potential for extensive epidemics.

Regarding coronaviruses with a human tropism in general and SARS CoV in particular, there is, to date, no vaccine, no prophylactic nor therapeutic treatment against this agent. Ribavirin and interferon have been used in the absence of other candidate but their intrinsic efficiency against the SARS CoV appears to be modest.

Coronaviruses have been characterized as having a very strong tropism in animals and represent an important threat in agriculture and domestics animal. This includes that:
- the possibility to get a treatment against these viruses, when no vaccines are available, opens a new area in the management of these highly costly diseases.
- one cannot eliminate the possibility of a new emerging Coronavirus. The possibility of targeting an enzyme from these viruses represents a valuable tool in the management of the disease in animals, when it has already been described with SARS that virus can escape its original host to infect humans with an incredible efficiency.

By analogy with existing antiviral therapies (e.g. against HIV), the replication machinery of RNA viruses (e. g. coronaviruses) can be regarded as a target of choice for the search of antiviral drugs.

However, in the case of Coronaviruses, any viral RNA polymerase activity has never been demonstrated despite the fact that one protein (nsp12 in the SARS CoV nomenclature) contains signature sequences typical of RNA dependent RNA polymerases. In fact, most proteins of the replicative machinery of coronaviruses are still of unknown function. It is thus urgent to discover viral targets that can be characterized and be the object of inventive work so as to use them to find antiviral drugs. This endeavour is the subject of the present invention.

### Current state-of-the-art in drug-screening and drug-design.

So far, the main protease 3CLPro is the subject of intense efforts to screen and discover potential antiviral molecules. Several research groups in the world are actively and successfully working on this area, and are proposing new molecules as potential antivirals. Clearly, alternative viral targets are needed to expand the capacity of finding new drugs. The replicative machinery (e.g., the RNA polymerase) is not only attractive in itself, but also because RNA polymerase inhibitor could be combined to an antiprotease inhibitor in bi-therapy treatments.

### Replicase and replication cycle

Coronaviruses are enveloped, positive-stranded RNA (+RNA) viruses, with a single-stranded genome of between 27 and 31.5 kilobases, the largest genome among known RNA viruses. The coronavirus replicase polyproteins contain a group of largely uncharacterised replicative enzymes, which drive viral RNA synthesis and genome expression.

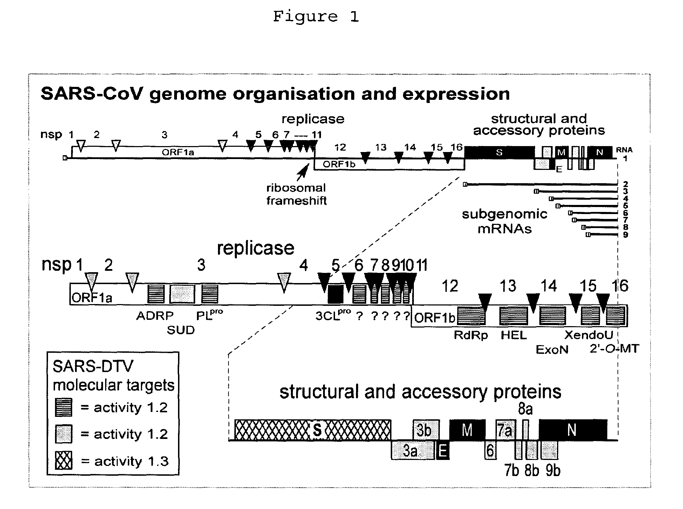

The coronavirus pp1a and pp1ab precursors are cleaved into functional subunits by two or three ORF1a-encoded viral proteinases (two in the case of SARS-CoV). The mature non-structural proteins assemble into a viral replication complex that is targeted to cytoplasmic membranes. The SARS-CoV genome is -29.7 kilobases long and contains 14 open reading frames (ORFs) that are flanked by 5'- and 3'-untranslated regions of 265 and 342 nucleotides, respectively, as disclosed in Figure 1 which represents the SARS-CoV genome organisation and genome expression. Homologues of replicative domains conserved in all coronaviruses are found in the pp1a and pp1ab non-structural polyproteins, whereas the S, M, E, and N proteins are encoded by ORFs 2, 5, 6 and 9a, respectively.

### The RNA polymerase activity, a validated viral target, is missing in coronaviruses

Based on signature-sequences, a putative RNA polymerase activity has been identified using computer-based motif searches to one protein, nsp12 in the SARS CoV nomenclature. Since the viral RNA polymerase is critical for the replication of the virus and cannot be substituted by any other cellular polymerase, it is an excellent antiviral target.

This explains why, for other viruses, most of more than 30 new antiviral agents, which have been developed and approved during the last 5 years, are directed against viral polymerases. They are mainly targeted against human immunodeficiency virus, but drugs against hepatitis B and C, herpes simplex, varicella-zoster and influenza virus infections have also been made commercially available. More than 50% of them are nucleoside analogues, in which either the base or the ribose moiety or both have been modified.

The major way of drug discovery today is to identify new antiviral agents through high-throughput screening (HTS) of a high number of synthetic or natural compounds. This requires the development of an in vitro assay, which in turn requires large amounts of soluble and active protein.

Many antiviral compounds have been discovered using cell cultures infected with the virus of interest. In this case, the addition of an antiviral compound protects the cells from infection, or inhibits virus growth. When a high number of such compounds are tested by HTS, it is possible to identify such antiviral compounds of interest. This approach has been used successfully for HIV or other viruses. In some cases, this approach is difficult due to the absence of a suitable system allowing infection of a cell in vitro. In other cases, even if a suitable cell-based assay is available, this procedure may be too cumbersome or expensive. This is the case for dangerous viruses for which a BL-4 facility is required. Setting up a complete screening process of million of compounds into a BL-4 containment has not been achieved yet. In such cases, it is preferable to screen such compounds directly on viral target proteins. Coronaviruses belong to this class of viruses. They require from BSL-3 to BSL-4 facilities (e.g., SARS Human Coronavirus).

The characterization in molecular terms of the target, the viral polymerase, is thus of prime importance to be able to screen and select antiviral compounds. In the case of the Coronavirus RNA polymerase, expression and purification of the RNA polymerase gene has not been reported yet. Polymerase genes have been notoriously difficult to clone in their entirety. Many recombinant polymerase genes have been reported to be unstable in bacterial hosts. In addition, the notoriously low yield of soluble purified RNA polymerases is a limiting factor to set up polymerase-activity assays.

RNA polymerases are generally error prone, and the large coronavirus genome would be likely prone to many mutations at each replication cycle. To avoid such deleterious high mutation rates, large DNA viral genomes such as those of bacteriophages T4, T7 have evolved with a proof-reading activity able to correct errors. Other systems such as eukaryotic/prokaryotic cells have evolved toward the selection of specific repair systems, in which several polymerases are involved (replicative polymerases and repair polymerases). To date, no viral RNA genomes are known to carry more than one polymerase gene. It is obvious that a second auxiliary yet essential polymerase would also be a good target for antiviral drugs.

With no prior knowledge, and solely as a result of a systematic search of novel enzymatic activities suitable for drug-design, the inventors have discovered a novel RNA polymerase activity. In this invention, the inventors report the discovery and identification of a novel polymerase gene encoded by the gene product nsp8 of the ORF 1a/1ab of the SARS CoV. They have purified nsp8 and shown that it is an RNA dependent RNA polymerase. The nsp8 gene has been localized in many of the members of the *Nidovirales* virus family. The inventors have evaluated the potential of this novel enzyme as a target for drug-design. They have found that the activity can be inhibited by nucleotide analogues, is able to perform *de novo* RNA synthesis, and is suitable as a catalyst to perform RNA synthesis *in vitro.*

Thus, the inventors have identified a novel RNA dependent RNA polymerase from the *Nidovirales* family, demonstrated its activity and its use in *vitro* as a tool in molecular biology and drug target for drug screening against *Nidovirales,* which is the main subject of the present invention.

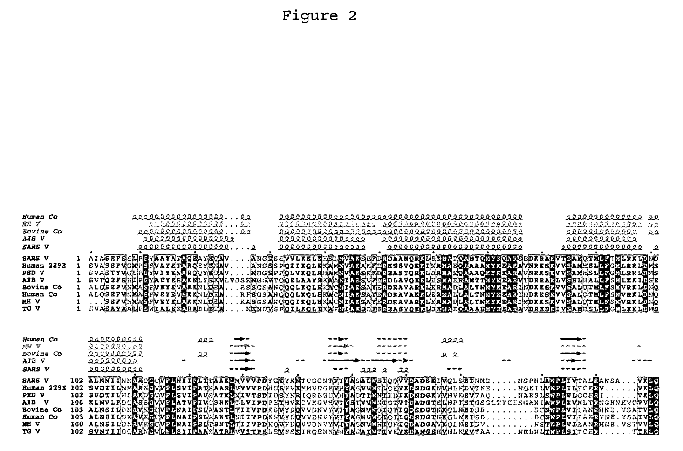

Moreover, the inventors have identified that the nsp8 protein is highly conserved among the coronaviruses as shown by the analysis of the comparison of the sequence of the NSP8 genes from the different Coronaviruses (see Figure 2). This implies that it can be anticipated that the function of the product of the NSP8 genes is going to be a polymerase, and that the demonstrated findings in this application apply to other *Nidovirales,* and potentially to all novel viruses possessing such homologous gene for which RNA polymerase can be demonstrated.

Thus, the present invention concerns a RNA dependent RNA polymerase the amino acid sequence of which presents at least 60% similarity with the following sequence :
AIASEFSSLPSYAAYATAQEAYEQAVANGDSEVVLKKLKKSLNVAKSEFDRDAA MQRKLEKMADQAMTQMYKQARSEDKRAKVTSAMQTMLFTMLRKLDNDALNNIINNARDGC VPLNIIPLTTAAKLMVVVPDYGTYKNTCDGNTFTYASALWEIQQVVDADSKIVQLSEINM DNSPNLAWPLIVTALRANSAVKLQ (SEQ ID NO. 1 in the sequence listing in appendix) and fragments thereof.

By « fragment », it must be understood a fragment of the RNA dependent RNA polymerase which goes on presenting a polymerase activity. The one skilled in the art has sufficient information hereinafter (polymerase assay given in the examples) to identify said fragments without undue experimentation. Preferably, said fragments present a sequence of 50, 60, 70, 80, 90, 100, 110, 120, 130, 140, 150, 160, 170, 180, 190 or 200 amino acids.

In a preferred embodiment, the amino acid sequence of the RNA dependent RNA polymerase subject-matter of the invention presents 65, 70, 75, 80, 85, 90, 95, or 100% similarity with the sequence represented by SEQ ID NO. 1.

Advantageously, the RNA dependent RNA polymerase subject-matter of the present invention is chosen in the group comprising :
AIASEFSSLPSYAAYATAQEAYEQAVANGDSEWLKKLKKSLNVAKSEFDRDAA MQRKLEKMADQAMTQMYKQARSEDKRAKVTSAMQTMLFTMLRKLDNDALNNIINNARDGC VPLNIIPLTTAAKLMVVVPDYGTYKNTCDGNTFTYASALWEIQQVVDADSKIVQLSEINM DNSPNLAWPLIVTALRANSAVKLQ (ORF1ab polyprotein SARS coronavirus - isolate TOR2 ; SEQ ID NO. 1 in the sequence listing in appendix),
SVTQEFSHIPSYAEYERAKNLYEKVLVDSKNGGVTQQELAAYRKAANIAKSVFD RDLAVQKKLDSMAERAMTTMYKEARVTDRRAKLVSSLHALLFSMLKKIDSEKLNVLFDQA SSGVVPLATVPIVCSNKLTLVIPDPETWVKCVEGVHVTYSTVVWNIDTVIDADGTELHPT STGSGLTYCISGANIAWPLKVNLTRNGHNKVDVVLQ (ORF1ab polyprotein Avian infectious bronchitis virus [Beaudette] ; SEQ ID NO. 2 in the sequence listing in appendix),
ALQSEFVNMASFVEYEVAKKNLDEARSSGSANQQQLKQLEKACNIAKSAYERDR AVARKLERMADLALTNMYKEARINDKKSKVVSALQTMLFSMVRKLDNQALNSILDNAVKG CVPLNAIPSLAANTLTIIVPDKSVYDQVVDNVYVTYAGNVWQIQTIQDSDGTNKQLNEIS DDCNWPLVIIANRHNEVSATVLQ (ORF1ab polyprotein Bovine coronavirus - isolate BCoV-ENT ; SEQ ID NO. 3 in the sequence listing in appendix),
SVASSFVGMPSFVAYETARQEYENAVANGSSPQIIKQLKKAMNVAKAEFDRESS VQKKINRMAEQAAAAMYKEARAVNRKSKVVSAMHSLLFGMLRRLDMSSVDTILNMARNGV VPLSVIPATSAARLVVVVPDHDSFVKMMVDGFVHYAGVVWTLQEVKDNDGKNVHLKDVTK ENQEILVWPLILTCERVVKLQ (ORF1ab polyprotein Human coronavirus 229^{E}; SEQ ID NO. 4 in the sequence listing in appendix),
ALQSEFVNMASFVEYEVAKKNLDEARFSGSANQQQLKQLEKACNIAKSAYERDR AVAKKLERMADLALTNMYKEARINDKKSKVVSALQTMLFSMVRKLDNQALNSILDNAVKG CVPLNAIPSLAANTLNIIVPDKSVYDQVVDNVYVTYAGNVWQIQTIQDSDGTNKQLNEIS DDCNWPLVIIANRYNEVSATVLQ (polyprotein lab Human coronavirus [ATCC VR-759] - isolate OC43 ; SEQ ID NO. 5 in the sequence listing in appendix),
SEFVNMASFVEYELAKKNLDEAKASGSANQQQIKQLEKACNIAKSAYERDRAVA RKLERMADLALTNMYKEARINDKKSKVVSALQTMLFSMVRKLDNQALNSILDNAVKGCVP LNAIPSLTSNTLTIIVPDKQVFDQVVDNVYVTYAGNVWHIQFIQDADGAVKQLNEIDVNS TWPLVIAANRHNEVSTWLQ (ORF1ab polyprotein Murine hepatitis virus [MHV-A59] ; SEQ ID NO. 6 in the sequence listing in appendix),
SVASTYVGLPSYVIYENARQQYEDAVNNGSPPQLVKQLRHAMNVAKSEFDREAS TQRKLDRMAEQAAAQMYKEARAVNRKSKVVSAMHSLLFGMLRRLDMSSVDTILNLAKDGV VPLSVIPAVSATKLNIVTSDIDSYNRIQREGCVHYAGTIWNIIDIKDNDGKVVHVKEVTA QNAESLSWPLVLGCERIVKLQ (polyprotein Porcine epidemic diarrhea virus [CV777] ; SEQ ID NO. 7 in the sequence listing in appendix),
SVASAYAALPSWIALEKARADLEEAKKNDVSPQILKQLTKAFNIAKSDFEREAS VQKKLDKMAEQAAASMYKEARAVDRKSKIVSAMHSLLFGMLKKLDMSSVNTIIDQARNGV LPLSIIPAASATRLVVITPSLEVFSKIRQENNVHYAGAIWTIVEVKDANGSHVHLKEVTA ANELNLTWPLSITCERTTKLQ (ORF1ab polyprotein Transmissible gastroenteritis virus [Purdue] - isolate PUR46-MAD ; SEQ ID NO. 8 in the sequence listing in appendix).

With the information disclosed in the present invention, the one skilled in the art has sufficient information (the above sequences and the polymerase assay given in the examples) to identify additional RNA dependent RNA polymerases from the *Nidovirales* family. Moreover, proteins presenting modifications such as amino acid insertion, deletion or substitution but still presenting a RNA polymerase activity are in the scope of the present invention.

The invention concerns a nucleic acid molecule comprising or constituted of an encoding nucleic sequence for a RNA dependent RNA polymerase as above-disclosed. Evidently, the invention also concerns nucleotide sequences derived from the above sequences, for example from the degeneracy of the genetic code, and which encode for proteins presenting characteristics and properties of the RNA dependent RNA polymerases of the invention.

Another aim of the present invention is polyclonal or monoclonal antibodies directed against a polypeptide of the invention, a derivative or a fragment of these. These antibodies can be prepared by the methods described in the literature. These antibodies are useful in the search for new RNA dependent RNA polymerases or the homologues of this enzyme in other virus belonging to the *Nidovirales* family.

The invention also concerns a vector comprising at least one molecule of nucleic acid above, advantageously associated with adapted control sequences, together with a production or expression process of the present RNA dependent RNA polymerase or a fragment thereof, either alone or fused to any sequence, in a cellular host. The subject-matter RNA polymerase gene can be fused to any other expressed sequence, and serve as an activity tag for localizing the whole construct in the host cell. The subject-matter RNA polymerase activity serves as a reporter in host cells in which the vector is introduced. Detection of RNA polymerase activity gives an indication of fonctionality of the gene fusion, as well as of localization of the whole construct in the host cell. The sensitivity of the subject-matter polymerase to inhibitors such as 3'-deoxy NTP allows to switch off the activity of the subject-matter polymerase in the presence of 3'-deoxy NTP. The latter compounds can be made available inside the host cell by incubating the vector-containing host cells with 3'-deoxy nucleosides, the latter being activated to their 5'-triphosphate stage by the host cell cellular kinases.

The subject-matter RNA dependent RNA poymerase can also be used to measure of NTP pools *in situ,* as well as the localization of polymerization foci to localize a gene construct fused to the present RNA polymerase. The preparation of these vectors as well as the production or expression of the proteins subject-matter of the present invention in a host can be carried out by molecular biology and genetic engineering techniques well known to the one skilled in the art.

An encoding nucleic acid molecule for an RNA dependent RNA polymerase or a vector according to the invention can also be used to transform cells and/or animals and establish a line of transgenic cells and/or animals. The vector used is chosen in function of the host into which it is to be transferred; it can be any vector such as a plasmid, a cosmid, or any other nucleic acid vector. Thus the invention also relates to transgenic cell transformed by a molecule of nucleic acid or by a vector according to the present invention expressing a polypeptide subject-matter of the present invention obtained in conformity with the preceding processes. More particularly, said cellular hosts are cells from insects, from mammalians, preferably from non-human mammalians such as rat, mouse, pig, cow, dog, cat, or any relevant host. Alternatively, the transgenic animals subject-matter of the present invention which comprise at least one transgenic cell according to the present invention can be insects, mammalians, preferably non-human mammalians such as rat, mouse, pig, cow, dog, cat, or any relevant host.

The invention also relates to nucleic and oligonucleotide probes prepared from the molecules of nucleic acid according to the invention. These probes, marked advantageously, can contain between 15 and 100 nucleotides, preferable between 15 and 50 nucleotides and, more preferably, between 15 and 30 nucleotides. Said nucleic and oligonucleotide probes are useful for hybridisation detection of RNA dependent RNA polymerases in other virus belonging to the *Nidovirales* family. According to prior art techniques, these probes are put into contact with a biological sample. Different hybridisation techniques can be used, such as Dot-blot hybridisation or replica hybridisation (Southern technique) or other techniques (DNA chips). Such probes constitute the tools making it possible to detect similar sequences quickly in the encoding genes of different virus belonging to the *Nidovirales* family. The oligonucleotide probes are useful for PCR experiments, for example in a diagnostic aim.

The present invention also concerns the use of the RNA dependent RNA polymerase subject-matter as a biotech reagent, more particularly, as a molecular biology reagent and, preferably, as for transcription based amplification methods. Generally, transcription based amplification methods involved the transcription of multiple RNA copies from a RNA template comprising a promoter recognized preferably by an RNA polymerase. The subject-matter RNA polymerase is able to synthesize RNA in a promotor- and primer-independent fashion. Thus, the RNA dependent RNA polymerase subject-matter can be used to synthesize RNA preferably using as starting material an RNA, according to the methods known by the one skilled in the art. The RNA synthesis driven by the subject-matter polymerase can be used for many purposes in molecular biology, such as RNA sequencing. In this use, RNA chain-termination inhibitors, such as 3'-deoxy GTP, 3'-deoxy ATG, 3'-deoxy UTP, and 3'-deoxy CTP, can be incorporated in the elongating RNA molecule to produce a nested set of RNAs useful in the determination of the nucleotide sequence of the template RNA. The technique of sequence determination is analogous to that described for DNA sequencing by the well-known Sanger's method.

The present invention can also be useful in a method for screening biologically active compound which can be used as antiviral agent. Preferably, said method comprises the following steps :
a) incubating selected different concentrations of said biologically active compound with the RNA dependent RNA polymerase subject-matter of the present invention,
b) quantifying the RNA dependent RNA polymerase activity,
c) comparing said activity with the RNA dependent RNA polymerase activity in absence of the compound.

It is clear that a biologically active material which can diminish or inhibit said activity will be a good candidate as antiviral agent and more particularly against viruses belonging to the *Nidovirales* family. The one skilled in the art knows different methods to quantify the RNA dependent RNA polymerase activity. Such a method is disclosed in the following examples (see example II).

Thus, the activity can be measured by the formation of homopolymeric (e.g., poly G, poly A, U,...) or heteropolymeric RNA in presence of an RNA template and appropriate radioactive nucleotides and, more particularly, labelled on their alpha phosphate. The label is incorporated into RNA products that are absorbed as insoluble radio-labeled material onto filter papers. The unbound, unincorporated label is washed away, and RNA synthesis products are quantitated using liquid scintillation counting. Alternatively, RNA synthesis products can be analyzed using denaturing gel electrophoresis, or any other existing method using radiolabeled or non-radioactive reagents easily adapted to this polymerase assay by the one skilled in the art.

Other advantages and characteristics of the invention will become apparent by reading the following examples concerning the cloning of the SARS nsp8 gene, the expression and purification of it gene product nsp8, its activity and inhibition of said activity by nucleotide analogues, and in which:
Figure 1 represents the SARS-CoV genome organisation and genome expression, showing the previously uncharacterized nsp8 protein.
Figure 2 represents the SDS-PAGE analysis of purified nsp8 (2µg) ;
Figure 3 concerns a graphic representation of incorporated nucleoside monophosphates into RNA by nsp8. PolyC without nsp8 : no activity detected. PolyC+nsp8 activity detected. PolyC+nsp8+Rifampicine (an inhibitor of the bacterial polymerase) : activity detected, the activity is not linked to a bacterial contamination of the nsp8 sample. Poly A and Poly U : no activity detected with other template ;
Figure 4 represents the visualisation of the nsp8 polymerase activity on a denaturing polyacrylamide gel ;
Figure 5 represents the RNA polymerase assay of nsp8 and its inhibition by 3'-deoxyGTP;
Figure 6 represents the determination of the IC50 of 3'-deoxy GTP towards nsp8.

### I. Cloning of the SARS nsp8 gene, and expression and purification of its gene product nsp8.

SARS genomic RNA was isolated from the Frankfurt isolate. Parts of the genome were reverse transcribed and amplified into cDNA (RT-PCR). SARS cDNAs were a kind gift of Dr. E. M. Snijder, LUMC, Leiden, the Netherlands.

The SARS-CoV Nsp8-coding sequence was amplified by PCR from the SARS cDNA using two primers containing the attB sites of the Gateway recombination system (Invitrogen). At the 5' end of the gene was attached a sequence encoding a hexahistidine tag. The amplified product was then sub-cloned into the pDest14 plasmid (Invitrogen).

The correctness of the open reading frame of the final construct (referred to as pDest14/Nsp8-HN) and encoding an N-terminally His-tagged version of SARS-CoV replicase residues (3920-4117) was checked by DNA sequencing. Expression was carried out in the E. *coli* strain C41(DE3) (Avidis S.A., France) transformed with the pLysS plasmid (Novagen). Cultures were grown at 37°C untill OD600 reached 0.6, expression was then induced by adding 0.5 mM IPTG and incubation was continued for 4-5 hours at 37°C. Cells were collected by centrifugation and the bacterial pellets were resuspended and frozen in 50 mM Tris-HCl, 150 mM NaCl, 10 mM imidazole pH 8.0. Cellular suspensions were thawed with 0.25 mg/mL lysozyme, 0.1 µg/mL DNase and 20 mM MgSO4 and centrifuged at 12,000 g. The supernatant was applied onto a Ni affinity column connected to a FPLC system. The protein was eluted in 50 mM Tris-HCl, 150 mM NaCl, 250 mM imidazole, pH 8.0 and then applied onto a preparative Superdex 200 gel filtration column pre-equilibrated in 10 mM Tris-HCl, 300 mM NaCl, pH 8.0.

The figure 2 presents the SDS-PAGE analysis of the purified nsp8. Said protein is a 22 kDa protein.

### II. Activity of the SARS nsp8 protein.

Polymerase activity was assayed using a filter paper assay. In this assay, radioactive nucleotides labelled on their alpha phosphate are incorporated into RNA synthesized by nsp8. The product of the reaction are spotted onto a filter paper, the unincorporated nucleotides are washed away, and the neo-synthesized radioactive RNAs remain on the filters which are subsequently analyzed by liquid scintillation counting.

The assay is described in details for the incorporation of GMP into a poly G RNA using a polyC template, but it is easily transposable for the synthesis of any homopolymeric (e.g., poly A, U,...) or heteropolymeric RNA.

### II.1. Description of the assay.

RNA polymerization kinetic of nsp8 in the presence of polymerization buffer (PB, made of 50mM Tris pH7,5; 10mM KCl; 4mM MgCl₂; 1mM MnCl₂; 10µg/ml BSA; 0,5% Triton; 10mM DTT), 0,005µCi [³H] GTP, and 10µM GTP. The template is an homopolymeric RNA (1µM polyC). In one assay, Rifampicine is present at 40µg/µl.

Reactions are incubated at 30°C in a final volume of 20 to 100 µl. Aliquots are withdrawn and spotted onto Whatman DE-81 paper circles, washed 3 times with 0.3 M ammonium formate pH 8.0, 2 times with ethanol, dried, and analysed using liquid scintillation counting.

### II.2. Results.

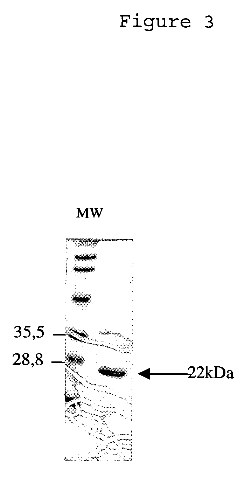

The following table summarises the results obtained and the figure 3 shows the plotting of these data.

| **Time(min).** | **polyC** | **polyC+nsp8** | **polyC+nsp8 +Rifampicine** | **polyA** | **polyA+nsp8** | **polyU** | **polyU+nsp8** |
|---|---|---|---|---|---|---|---|
| 0 | 126 | 559 | 1486 | 67 | 98 | 100 | 127 |
| 1 | 76 | 2989 | 3540 | 67 | 68 | 128 | 153 |
| 2 | 108 | 6209 | 7731 | 51 | 67 | 144 | 161 |
| 5 | 63 | 13806 | 16777 | 69 | 55 | 122 | 188 |
| 10 | 81 | 29690 | 31547 | 58 | 69 | 148 | 197 |
| 15 | 87 | 41759 | 50196 | 69 | 72 | 128 | 273 |
| 60 | 65 | 80219 | 83510 | 47 | 78 | 112 | 2092 |

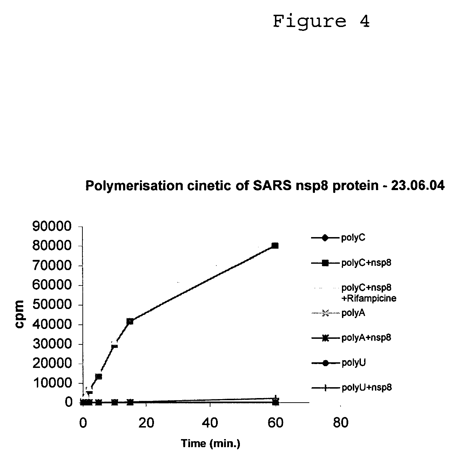

Such a polymerase assay can be visualized on a denaturing polyacrylamide gel, as shown on figure 4.

It is clear that nsp8 can synthesize an RNA product, without a primer, up to a 5-mer product, then extend this product up to very high molecular weight products under a very processive mode of RNA synthesis. In addition to the obvious use such a novel reagent can provide in the search of antival drugs, the capability of nsp8 to synthesize RNA so efficiently, together with the manageability of nsp8 in terms of ease of expression, high production yield, ease of purification, small molecular weight of an active enzyme make it an interesting novel molecular reagent for the life sciences.

For example, the nsp8 gene could be fused to any gene on a vector, and the whole could be used to transfect cells. Because endogenous RNA is generally found in the cell, the nsp8 activity could then be used *in situ* to monitor NTP polymerization. The sensitivity of the subject-matter polymerase to inhibitors such as 3'-deoxy NTP allows to switch off the activity of the subject-matter polymerase in the presence of 3'-deoxy NTP. The latter compounds can be made available inside the host cell by incubating the vector-containing host cells with 3'-deoxy nucleosides, the latter being activated to their 5'-triphosphate stage by the host cell cellular kinases.

Potential other applications are the measure of NTP pools *in situ,* as well as the localization of polymerization foci to localize a gene construct fused to nsp8.

### III. Nsp8 activity inhibition in presence of 50 µM 3'-deoxy GTP.

Since nsp8 is an RNA polymerase, such an assay can be used to monitor the inhibition of nsp8 by nucleotide analogues that are known to be RNA dependent RNA polymerase inhibitors. Such a well known compound is 3'-deoxy-GTP, and it was used to demonstrate that the activity of nsp8 can indeed be inhibited by such compounds.

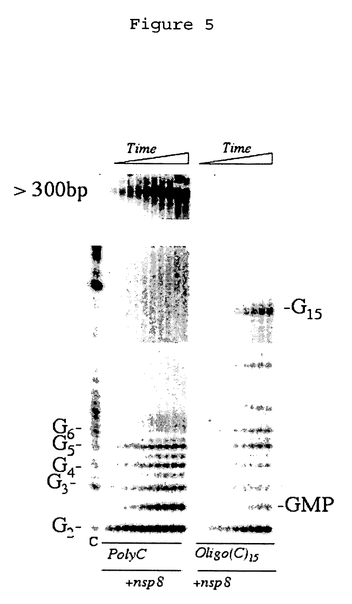

The experimental conditions as above were used for this assay, which is represented graphically in Figure 5.

The results clearly show that the RNA polymerase of nsp8 can be inhibited by 3'-deoxy GTP.

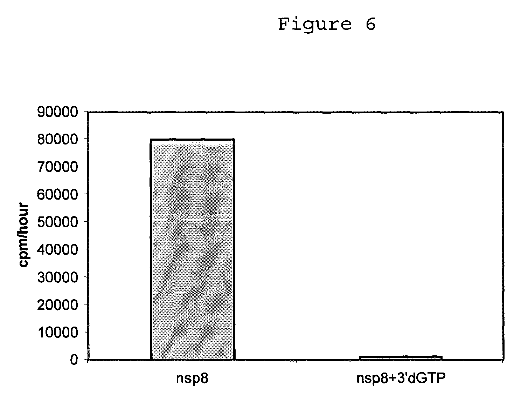

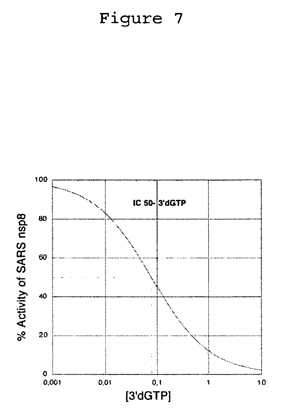

The precise IC50 of 3'-deoxy GTP, around 80 nM under these conditions, can be determined using standard methods of antiviral research, as shown below in Figure 6. Such an assay validate the use of nsp8 as a reagent usefull for drug-design of anticoronavirus drugs.

## Claims

1. A RNA dependent RNA polymerase the amino acid sequence of which presents at least 60% similarity with the following sequence :
AIASEFSSLPSYAAYATAQEAYEQAVANGDSEWLKKLKKSLNVAKSEFDRDAA MQRKLEKMADQAMTQMYKQARSEDKRAKVTSAMQTMLFTMLRKLDNDALNNIINNARDGC VPLNIIPLTTAAKLMVVVPDYGTYKNTCDGNTFTYASALWEIQQVVDADSKIVQLSEINM DNSPNLAWPLIVTALRANSAVKLQ (SEQ ID NO. 1 in the sequence listing in appendix) and fragments thereof.

2. RNA dependent RNA polymerase according to claim 1, which is chosen in the group comprising :
AIASEFSSLPSYAAYATAQEAYEQAVANGDSEWLKKLKKSLNVAKSEFDRDAA MQRKLEKMADQAMTQMYKQARSEDKRAKVTSAMQTMLFTMLRKLDNDALNNIINNARDGC VPLNIIPLTTAAKLMVVVPDYGTYKNTCDGNTFTYASALWEIQQVVDADSKIVQLSEINM DNSPNLAWPLIVTALRANSAVKLQ (ORF1ab polyprotein SARS coronavirus - isolate TOR2 ; SEQ ID NO. 1 in the sequence listing in appendix),
SVTQEFSHIPSYAEYERAKNLYEKVLVDSKNGGVTQQELAAYRKAANIAKSVFD RDLAVQKKLDSMAERAMTTMYKEARVTDRRAKLVSSLHALLFSMLKKIDSEKLNVLFDQA SSGVVPLATVPIVCSNKLTLVIPDPETWVKCVEGVHVTYSTVVWNIDTVIDADGTELHPT STGSGLTYCISGANIAWPLKVNLTRNGHNKVDVVLQ (ORF1ab polyprotein Avian infectious bronchitis virus [Beaudette] ; SEQ ID NO. 2 in the sequence listing in appendix),
ALQSEFVNMASFVEYEVAKKNLDEARSSGSANQQQLKQLEKACNIAKSAYERDR AVARKLERMADLALTNMYKEARINDKKSKVVSALQTMLFSMVRKLDNQALNSILDNAVKG CVPLNAIPSLAANTLTIIVPDKSVYDQVVDNVYVTYAGNVWQIQTIQDSDGTNKQLNEIS DDCNWPLVIIANRHNEVSATVLQ (ORF1ab polyprotein Bovine coronavirus - isolate BCoV-ENT ; SEQ ID NO. 3 in the sequence listing in appendix),
SVASSFVGMPSFVAYETARQEYENAVANGSSPQIIKQLKKAMNVAKAEFDRESS VQKKINRMAEQAAAAMYKEARAVNRKSKVVSAMHSLLFGMLRRLDMSSVDTILNMARNGV VPLSVIPATSAARLVVVVPDHDSFVKMMVDGFVHYAGVVWTLQEVKDNDGKNVHLKDVTK ENQEILVWPLILTCERWKLQ (ORF1ab polyprotein Human coronavirus 229^{E}; SEQ ID NO. 4 in the sequence listing in appendix),
ALQSEFVNMASFVEYEVAKKNLDEARFSGSANQQQLKQLEKACNIAKSAYERDR AVAKKLERMADLALTNMYKEARINDKKSKVVSALQTMLFSMVRKLDNQALNSILDNAVKG CVPLNAIPSLAANTLNIIVPDKSVYDQVVDNVYVTYAGNVWQIQTIQDSDGTNKQLNEIS DDCNWPLVIIANRYNEVSATVLQ (polyprotein lab Human coronavirus [ATCC VR-759] - isolate OC43 ; SEQ ID NO. 5 in the sequence listing in appendix),
SEFVNMASFVEYELAKKNLDEAKASGSANQQQIKQLEKACNIAKSAYERDRAVA RKLERMADLALTNMYKEARINDKKSKVVSALQTMLFSMVRKLDNQALNSILDNAVKGCVP LNAIPSLTSNTLTIIVPDKQVFDQVVDNVYVTYAGNVWHIQFIQDADGAVKQLNEIDVNS TWPLVIAANRHNEVSTVVLQ (ORF1ab polyprotein Murine hepatitis virus [MHV-A59] ; SEQ ID NO. 6 in the sequence listing in appendix),
SVASTYVGLPSYVIYENARQQYEDAVNNGSPPQLVKQLRHAMNVAKSEFDREAS TQRKLDRMAEQAAAQMYKEARAVNRKSKVVSAMHSLLFGMLRRLDMSSVDTILNLAKDGV VPLSVIPAVSATKLNIVTSDIDSYNRIQREGCVHYAGTIWNIIDIKDNDGKVVHVKEVTA QNAESLSWPLVLGCERIVKLQ (polyprotein Porcine epidemic diarrhea virus [CV777] ; SEQ ID NO. 7 in the sequence listing in appendix),
SVASAYAALPSWIALEKARADLEEAKKNDVSPQILKQLTKAFNIAKSDFEREAS VQKKLDKMAEQAAASMYKEARAVDRKSKIVSAMHSLLFGMLKKLDMSSVNTIIDQARNGV LPLSIIPAASATRLVVITPSLEVFSKIRQENNVHYAGAIWTIVEVKDANGSHVHLKEVTA ANELNLTWPLSITCERTTKLQ (ORF1ab polyprotein Transmissible gastroenteritis virus [Purdue] - isolate PUR46-MAD ; SEQ ID NO. 8 in the sequence listing in appendix).

3. A nucleic acid molecule comprising or constituted of an encoding nucleic sequence for a RNA dependent RNA polymerase according to any of claims 1 or 2.

4. Polyclonal or monoclonal antibodies directed against a RNA dependent RNA polymerase according to any of claims 1 or 2, a derivative or a fragment of these antibodies.

5. Vector comprising at least one molecule of nucleic acid according to claim 3, advantageously associated with adapted control sequences.

6. Transgenic cell transformed by a molecule of nucleic acid according to claim 3 or by a vector according to claim 5 expressing a RNA polymerase RNA dependent according to any of claims 1 or 2.

7. Transgenic animal comprising at least one cell according to claim 6.

8. Nucleic and oligonucleotide probes prepared from a molecule of nucleic acid according to claim 3.

9. Use of the RNA dependent RNA polymerase according to any of claims 1 or 2 as a biotech reagent.

10. Use of the RNA dependent RNA polymerase according to any of claims 1 or 2 in a method for screening biologically active compound which can be used as antiviral agent.

11. Method for screening biologically active compound which can be used as antiviral agent. Preferably, said method comprises the following steps :
a) incubating selected different concentrations of said biologically active compound with the RNA dependent RNA polymerase subject-matter of the present invention,
b) quantifying the RNA dependent RNA polymerase activity,
c) comparing said activity with the RNA dependent RNA polymerase activity in absence of the compound.
